(19) Europäisches Patentamt
European Patent Office
Office européen des brevets

(11) Veröffentlichungsnummer: **0 341 649 A1**

(12) # EUROPÄISCHE PATENTANMELDUNG

(21) Anmeldenummer: **89108292.7**

(22) Anmeldetag: **09.05.89**

(51) Int. Cl.[4]: **C12P 17/18 , A61K 31/00 , C07D 493/20 , //(C07D493/20, 307:00,303:00,303:00), (C12P17/18,C12R1:01)**

Der Anmelder hat eine Erklärung nach Regel 28 (4) EPÜ (Herausgabe einer Probe nur an einen Sachverständigen) eingereicht. Eingangsnummer(n) der Hinterlegung(en): DSM 4151

Der (Die) Mikroorganismus (Mikroorganismen) ist (sind) bei "Deutsche Sammlung von Mikroorganismen" unter der (den) Nummer(n) DSM 4151hinterlegt worden.

(30) Priorität: **13.05.88 DE 3816411**

(43) Veröffentlichungstag der Anmeldung: **15.11.89 Patentblatt 89/46**

(84) Benannte Vertragsstaaten: **AT BE CH DE ES FR GB GR IT LI LU NL SE**

(71) Anmelder: **HOECHST AKTIENGESELLSCHAFT**
**Postfach 80 03 20**
**D-6230 Frankfurt am Main 80(DE)**

(72) Erfinder: **Roy, Kirity, Dr.**
**G-31, Hoechst Quarters Darga Road**
**Mulund (West) Bombay 400 082(IN)**
Erfinder: **Mukhopadhyay, Triptikumar, Dr.**
**M-24, Hoechst Quarters Darga Road**
**Mulund (West) Bombay 400 082(IN)**
Erfinder: **Reddy, Goukanapalli, Dr., Chandra Shekhara**
**104 Aarti Apartments Sarojini Naidu Road**
**Mulund (West) Bombay 400 080(IN)**
Erfinder: **Vijayakumar, Erra Koteswara Satya, Dr.**
**K-3, Hoechst Quarters Darga Road**
**Mulund (West) Bombay 400 080(IN)**
Erfinder: **Ganguli, Bimal Naresh, Dr.**
**Saurayuth 173 Central Avenue**
**Chembur Bombay 400 071(IN)**
Erfinder: **Rupp, Richard Helmut, Dr.**
**Roederweg 16a**
**D-6240 Köningstein/Taunus(DE)**
Erfinder: **Fehlhaber, Hans-Wolfram, Dr.**
**Thomas-Mann-Strasse 5a**
**D-6270 Idstein/Taunus(DE)**
Erfinder: **Kogler, Herbert, Dr.**
**Breslauer Strasse 39**
**D-6233 Kelkheim(Taunus)(DE)**

(54) **Ein neues Antibiotikum, Aranorosin, ein mikrobiologisches Verfahren zu seiner Herstellung und seine Verwendung als Arzneimittel.**

(57) Aranorosin, eine Verbindung der Formel

EP 0 341 649 A1

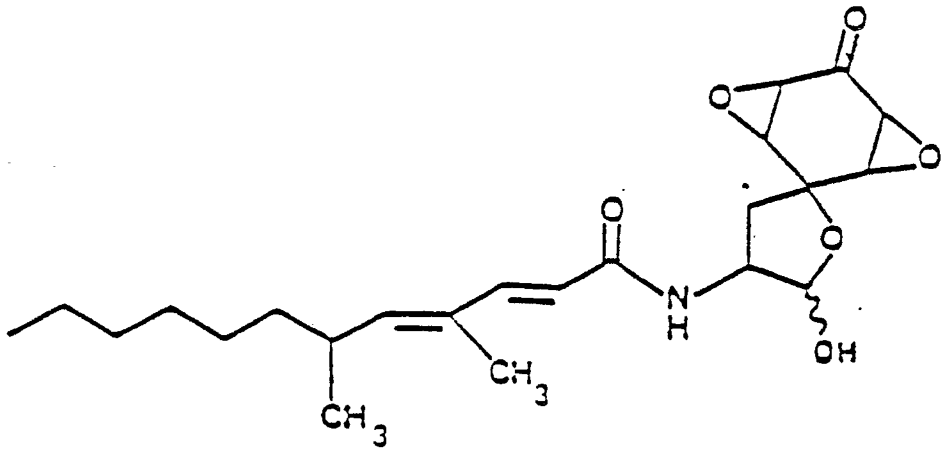

läßt sich mit Hilfe der Pilzkultur Y-30499 (DSM 4151) herstellen. Aranorosin hat antibiotische Wirkung sowie eine Wirkung gegen maligne Tumore.

# Ein neues Antibiotikum, Aranorosin, ein mikrobiologisches Verfahren zu seiner Herstellung und seine Verwendung als Arzneimittel

Die vorliegende Erfindung betrifft ein neues Antibiotikum, Aranorosin sowie ein Verfahren zu dessen Herstellung aus Pilzkultur Nr. Y-30499.

Die Pilzkultur Y-30499 zur Herstellung von Aranorosin wurde aus Boden isoliert und als Pseudoarachniotus roseus Kuehn (Mycologia 69; 126 - 163, 1977) identifiziert, der zur Familie Gymnoascaceae, Ordnung Eurotiales, Klasse Plectomycetes, Unterabteilung Ascomycotina und Abteilung Eumycota gehört (G.C. Ainsworth, F.K. Sparrow und A.S. Sussman (Herausgeber) 1973, "The Fungi, An Advanced Treatise" Band IVA und IVB, Academic Press, New York). In der Literatur sind keine durch diese Pilzkultur produzierte Antibiotika beschrieben.

Die Kultur Y-30499 wude am 23.06.1987 bei der "Deutsche Sammlung von Mikroorganismen", Göttingen, unter der Eingangsnummer DSM 4151 hinterlegt.

Aranorosin hat die folgende Strukturformel

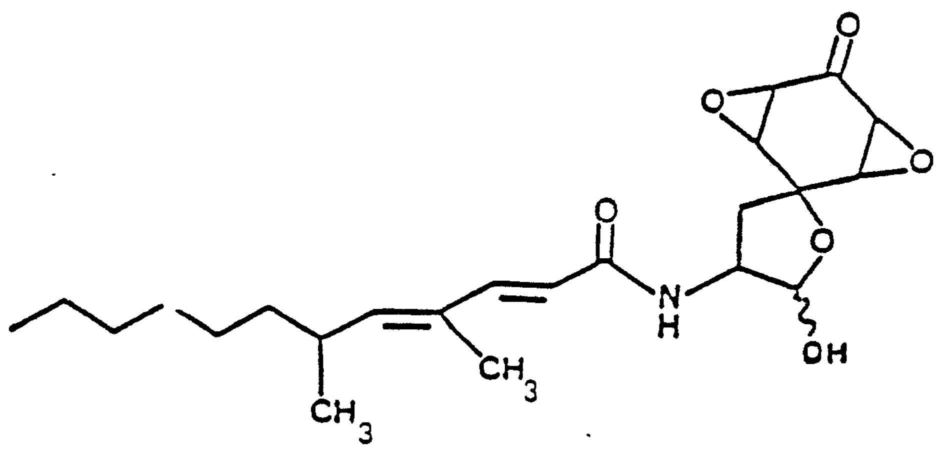

Gegenstand vorliegender Erfindung ist auch ein Verfahren zur Herstellung des neuen Antibiotikums Aranorosin aus der Pilzkultur Nr. Y-30499, welches darin besteht, daß man diese Pilzkultur durch Fermentation unter aeroben Bedingungen auf einem Nährboden, der Kohlenstoffquellen, Stickstoffquellen, anorganische Nährsalze und Spurenelemente enthält, bei einer Temperatur zwischen 24 und 30 °C und einem pH zwischen 6,0 und 8,0 66 bis 90 Stunden lang kultiviert und das Antibiotikum nach den üblichen Methoden aus der Kulturbrühe isoliert und reinigt.

Als Kohlenstoffquellen kommen Glucose, Rohrzucker, Stärke oder Dextrin in Frage. Stärke wird als Kohlenstoffquelle bevorzugt. Als Stickstoffquellen kommen Soyabohnenmehl, Trypton, Hefeextrakt, Rindfleischextrakt, Malzextrakt, Maisquellwasser, Pepton oder anorganische Stoffe wie Ammoniumsalze in Betracht. Die bevorzugte Stickstoffquelle ist Soyabohnenmehl. Bei den anorganischen Nährsalzen kann es sich um Natriumchlorid, Kaliumhydrogen- und -dihydrogenphosphat oder Calciumcarbonat handeln. Als Spurenelemente können Salze des Eisens, Mangans, Kupfers, Zinks oder Kobalts vorliegen.

Zweckmäßig wird die Züchtung der Kultur Nr. Y-30499 bei 26 °C (± 1 °C) und pH 6,5 durchgeführt. Die Kultivierung kann sowohl in Schüttelkolben als auch Fermentern erfolgen. Die Kultivierung erfolgt vorzugsweise 72 Stunden lang. Nach dieser Zeit erhält man offenbar die maximale Ausbeute des erfindungsgemäßen Antibiotikums. Die Kultivierung kann vorzugsweise submers erfolgen. Wird die Anzucht dieser Pilzkultur in Fermentern durchgeführt, so kann sie in Gegenwart eines Entschäumungsmittels wie eines linearen Polyäthers auf Propylenoxidgrundlage mit einem mittleren Molekulargewicht von 2000 ± 100 (Desmophen(R) der Firma Bayer AG) ablaufen.

Das Fortschreiten der Bildung des erfindungsgemäßen Aranorosins läßt sich durch Messung der Bioaktivität der Kulturbrühe gegen Bacillus subtilis und Aspergillus niger nach der bekannten Agarplatten-Diffusionsbestimmungsmethode erfassen.

Das erfindungsgemäße Aranorosin wird nach bekannten Standard-Laboratoriumsmethoden aus der Kulturbrühe isoliert und gereinigt. Zum Beispiel wird die Kulturbrühe zur Trennung des Mycels vom Kulturfiltrat zentrifugiert. Das Aranorosin läßt sich aus dem Kulturfiltrat durch Extraktion unter Verwendung eines mit Wasser nicht mischbaren Lösungsmittels wie Essigester, Chloroform oder Butanol nach Einstellung des pH des Filtrats auf 7,0 gewinnen; dabei wird Essigester als Extraktionsmittel bevorzugt. Aranorosin im Mycel wird gewonnen, indem man das Mycel mit einem Lösungsmittel wie Essigester, Chloroform, Methanol, Äthanol, Aceton oder Butanol extrahiert, wobei als Lösungsmittel Aceton bevorzugt ist. Nach der Extraktion entfernt man das Lösungsmittel durch Abdampfen im Vakuum, verdünnt die wäßrige Schicht mit Wasser und extrahiert nochmals mit einem Lösungsmittel wie den obigen. Die Lösungsmittelextrakte sowohl aus dem Kulturfiltrat als auch dem Mycel werden vereinigt, zur Trockne eingedampft und dann unter Anwendung von beispielsweise Säulenchromatographie gereinigt.

**Beispiel 1**

Stammhaltung der Kultur Nr. Y-30499

Die Kultur Nr. Y-30499 wird auf Glucoseagarmedium nach Sabouraud der folgenden Zusammensetzung unterhalten:

| | |
|---|---|
| Glucose | 40 g |
| Pepton | 10 g |
| $Na_2HPO_4$ | 1 g |
| Agar | 15 g |
| Destilliertes Wasser | 1 l |
| pH | 6,5 |

Nach vollständiger Auflösung der Bestandteile durch Erwärmen wird das Medium auf Reagenzgläser verteilt und dann 20 Minuten lang bei 121 °C sterilisiert. Der pH vor dem Autoklavieren beträgt 6,5. Die Reagenzgläser werden zur Herstellung von Schrägagars in Schrägstellung abgekühlt. Die Schrägagars werden mit Sporen der aus Boden isolierten Kultur Nr. Y-30499 beimpft und bei 26 °C (± 1 °C) inkubiert, bis gute Sporenbildung zu beobachten ist. Die Kulturen mit guter Sporenbildung werden im Kühlschrank aufbewahrt.

**Anzucht der Kultur Y-30499 in Schüttelkolben zur fermentativen Herstellung des neuen Antibiotikums Aranorosin.**

Zusammensetzung des Impfkulturmediums:

| | |
|---|---|
| Lösliche Stärke | 15 g |
| Soyabohnenmehl | 15 g |
| Glucose | 5 g |
| $CaCO_3$ | 2 g |
| NaCl | 5 g |
| Hefeextrakt | 2 g |
| Maisquellwasser | 1 g |
| Destilliertes Wasser | 1 l |

Je 100 ml des obigen Impfkulturmediums werden über weithalsige 500 ml-Erlenmeyerkolben verteilt und bei 121 °C 20 Minuten lang sterilisiert. Der pH wird vor dem Autoklavieren auf 6,5 eingestellt und beträgt danach 6,0. Die Kolben werden abgekühlt und dann mit einigen Platinösen voll der oben erwähnten Kultur mit guter Sporenbildung beimpft und 60 Stunden lang bei 240 U.p.M. bei 26 °C (± 1 °C) geschüttelt, wobei gutes Wachstum beobachtet wird. Die so erhaltene Impfkultur wird zum Beimpfen des Produktionsmediums der folgenden Zusammensetzung verwendet:

**Zusammensetzung des Produktionsmediums:**

| | |
|---|---|
| Soyabohnenmehl | 20 g |
| Glucose | 30 g |
| $CaCO_3$ | 6 g |
| NaCl | 3 g |
| $NH_4Cl$ | 1,5 g |
| $KH_2PO_4$ | 2 g |
| $ZnSO_4 \bullet 7H_2O$ | 0,22 mg |
| $CaCl_2$ | 0,55 mg |
| $MnCl_2 \bullet 4H_2O$ | 0,5 mg |
| $FeSO_4 \bullet 7H_2O$ | 0,5 mg |
| $CuSO_4 \bullet 5H_2O$ | 0,16 mg |
| $CoCl_2 \bullet 6H_2O$ | 0,16 mg |
| Destilliertes Wasser | 1 l |

Je 200 ml des obigen Produktionsmediums werden über 1 Liter-Erlenmeyerkolben verteilt und 20 Minuten lang bei 121 °C sterilisiert. Der pH des Mediums wird vor dem Autoklavieren auf 6,5 eingestellt und beträgt danach 6,0. Die Kolben werden abgekühlt und dann mit dem obigen Impfkulturmedium (1 % V/V) beimpft. Die Fermentation erfolgt 72 Stunden lang auf einer Rotationsschüttelmaschine bei 26 °C (± 1 °C). Die Aranorosinproduktion wird durch das Aktivitätsprofil gegen Bacillus subtilis und Aspergillus niger geprüft. Nach dem Ernten wird die Kulturbrühe zentrifugiert und das Aranorosin sowohl aus dem Mycel als auch dem Kulturfiltrat isoliert und wie unten beschrieben gereinigt.

**Isolierung und Reinigung des Aranorosins**

Das Kulturfiltrat (143 Liter, durch Zentrifugieren von ungefähr 150 Liter Kulturbrühe erhalten) wird bei pH 6,7 mit 50 Liter Essigester extrahiert. Die Extraktion wird nochmals wiederholt, und die vereinigten Extrakte werden von Essigester befreit und bei vermindertem Druck bei 35 °C eingeengt. Der Mycelkuchen (11,3 kg, durch Zentrifugieren von ungefähr 150 Liter Kultur erhalten) wird auf ähnliche Weise zweimal mit je 30 liter Aceton extrahiert. Der Großteil des Acetons wird bei vermindertem Druck bei 35 °C aus den kombinierten Extrakten entfernt, und die zurückbleibende wäßrige Phase wird zweimal mit je 3 Liter Essigester extrahiert. Die Essigesterextrakte werden bei vermindertem Druck bei 35 °C eingeengt und mit dem Konzentrat des Kulturfiltrierextrakts vereinigt.

Das rötlich braune rohe Antibiotikum Aranorosin (196 g) wird über Kieselgel (2 kg, 100 - 200 Maschen pro Zoll) chromatographiert und mit Chloroform/Methanolgemischen zunehmenden Methanolgehalts eluiert, bis 5 % Methanolkonzentration in Chloroform erreicht ist. Das Aranorosin wird zwischen 2 und 4 % Methanol in Chloroform eluiert.

Das halbreine Aranorosin (68 g) wird nochmals über Kieselgel (1,3 kg, 200 - 300 Maschen pro Zoll) chromatographiert und wie oben für die erste Säulenchromatographie erwähnt mit Chloroform/Methanolgemisch eluiert, wobei man weitergereinigtes Aranorosin (13 g) erhält.

Dieses Produkt wird wiederum über Kieselgel (650 g, 200 - 300 Maschen pro Zoll) mit Benzol/Acetonitrilgemisch (7 : 3) als Eluens chromatographiert. Reines Aranorosin (4,1 g) wird dabei als weißer Feststoff isoliert. Diesen löst man in Essigester und fällt zur Entfernung von Pigmentspuren mit Hexan aus.

**Beispiel 2**

**Anzucht der Kultur Nr. Y-30499 in Fermentern zur fermentativen Herstellung des neuen Antibiotikums Aranorosin**

**Stufe I: Herstellung der Impfkultur**

a) In Schüttelkolben:

Das Impfkulturmedium aus Beispiel 1 (100 ml) wird bei zuvor auf 6,5 eingestelltem pH in zuvor sterilisierten weithalsigen 500 ml-Erlenmeyerkolben vorgelegt, 20 Minuten lang im Autoklaven bei 121 °C sterilisiert, abgekühlt und mit einigen Platinösen voll der Kultur aus Beispiel 1 mit guter Sporenbildung beimpft. Der pH nach dem Sterilisieren beträgt 6,0. Die Kolben werden bei 26 °C (± 1 °C) 72 Stunden lang auf einer Rotationsschüttelmaschine bei 240 U.p.M. inkubiert.

b) In Saugflaschen:

Ein Liter des Impfkulturmediums aus Beispiel 1 wird in einer 5 Liter-Saugflasche vorgelegt, im Autoklaven 30 Minuten lang bei 121 °C sterilisiert, abgekühlt und mit einigen Platinösen voll der Kultur aus Beispiel 1 mit guter Sporenbildung beimpft. Der pH des Mediums beträgt 6,5 vor dem Sterilisieren und 6,0 danach. Die Flaschen werden in eine Rotationsschüttelmaschine gestellt und bei 26 °C (± 1 °C) 72 Stunden lang bei 240 U.p.M. inkubiert.

**Stufe II: Fermentation**

a) Im kleinen Maßstab:

Zehn Liter des Produktionsmediums aus Beispiel 1 werden mit 0,04 % Desmophen(R) in einem 15 Liter-Edelstahlfermenter 36 Minuten lang bei 121 - 122 °C autoklaviert. Der pH beträgt 6,5 vor dem Sterilisieren und 6,0 danach. Zwanzig Liter des Produktionsmediums aus Beispiel 1 mit 0,04 % "Desmophen(R)" werden in einem 30 Liter-Edelstahlfermenter vorgelegt und darin 32 Minuten lang bei 122 °C unter 1,2 kg/cm$^2$ Dampfdruck sterilisiert. Der pH wird vor dem Sterilisieren auf 6,5 eingestellt und beträgt danach 6,0. Nach dem Abkühlen wird der Fermenter mit 1 - 3 % (V/V) der Impfkultur unter aseptischen Bedingungen beimpft und bei 26 °C (± 1 °C) unter Rühren bei 120 - 180 U.p.M. je nach Wachstum und Schaumbildung sowie unter Belüftung mit 6 - 10 Liter pro Minute betrieben.

b) Im großen Maßstab

Einhundert Liter des Produktionsmediums aus Beispiel 1 mit auf 6,5 eingestelltem pH und 0,04 % Desmophen(R) werden in einem zuvor sterilisierten 150 Liter-Fermenter vorgelegt. Das Produktionsmedium wird darin bei 121 -122 °C 32 Minuten lang bei 1,2 kg/cm$^2$ Dampfdruck sterilisiert. Danach beträgt der pH 6,0. Nach Abkühlen auf 26 °C wird der Fermenter mit 1 % (V/V) der Impfkultur unter aseptischen Bedingungen beimpft und bei einer Temperatur von 26 °C (± 1 °C) unter Rühren bei 100 U.p.M. und mit 60 Liter pro Minute Belüftung betrieben.

Die Aranorosinproduktion wird durch das Aktivitätsprofil gegen Bacillus subtilis und Aspergillus niger geprüft. Die Fermenter werden nach 72 Stunden abgeerntet. Die Kulturbrühe wird zur Abtrennung des Mycels vom Kulturfiltrat zentrifugiert, und beide werden dann wie in Beispiel 1 beschrieben verarbeitet.

Das Aranorosin wurde durch chemische Analyse und spektroskopische Methoden untersucht. Es zeigt die folgenden Eigenschaften:

**Physikalische Eigenschaften des Antibiotikums Aranorosin**

| | |
|---|---|
| 1. Aussehen | Weißer Feststoff |
| 2. Summenformel | $C_{23}H_3NO_6$ |
| 3. Molekulargewicht | 419 |
| 4. Schmelzpunkt | 150 °C (Z) |
| 5. $[\alpha]_D^{25°}$ | -2,42° (c = 2,58 in Chloroform) |
| 6. Rf (Dünnschichtchromatographie auf Kieselgelfertigplatten) | 0,42 ( 85 : 15 Chloroform : Methanol) 0,39 (6 : 4 Benzol : Acetonitril) 0,49 (Essigester) |
| 7. Elementaranalyse | C 64,75; H 7,83; N 3,16 % |
| 8. Löslichkeit | Unlöslich in Petroläther, Wasser; schwerlöslich in Benzol; löslich in Diäthyläther, Chloroform, Aceton, Essigester, Dimethylsulfoxid und Methanol. |

**Spektral-Daten für das neue Antibiotikum Aranorosin**

UV (Methanol) λ max 264 nm IR (KBr) Bedeutende ν max. in $cm^{-1}$: 3448 (-OH und -NH), 1710 (6-gliedriges Ringketon), 1653 (sek. Amidcarbonyl, Bande I), 1538 (sek. Amidcarbonyl, Bande II), 1242 (Epoxid), 980 (trans-disubstituiertes Alken) und 844 (trisubstituiertes Alken)

$^1$H-NMR (270 MHz, $CDCl_3$, δ in ppm gegen TMS) 0,86 (t, J=6Hz, $-CH_3$), 0,95 (d, J=7Hz, $-CH_3$), 1,24 (s breit, 5X-$CH_2$), 1,75 (d, J=0,5Hz-=$CHCH_3$), 2,03 (dd, J=12,5,10,5Hz, COCHH), 2,48 (m,=CHCH), 2,60 (dd, J=12,5, 8,5Hz COCHH), 3,43 (m,CH-O-CH), 3,55 (dd, J=3,5, 3,5Hz, O-CH), 3,66 (dd, J=3,5Hz, O-CH), 4,26 (s breit, 1H, $D_2O$ austauschbar), 4,77 (m, NHCH), 5,61 (d, J=4,5Hz, CHOH), 5,65 (dd, J=10, 0,5Hz, =CH), 5,74 (d, J=15Hz, =CH), 6,06 (d, J=8Hz, -NH) und 7,23 (d, J=15Hz, =CH).

$^{13}$C-NMR (67,8 MHz, $CDCl_3$, δ in ppm gegen TMS) 12,47(q), 14,05(q), 20,49(q), 22,60(t), 27,44(t), 29,38-(t), $^{13}$C-NMR 31,81(t), 33,20(d), 35,96(t), 37,23(t), 52,00(d), 55,54(d), 55,78(d), 62,85(d), 64,21(d), 78,92-(s), 96,53(d), 116,85(d), 130,72(s) 147,33(d), 148,40(d), 166,67(s) und 198,19(s).

Die neue Verbindung Aranorosin besitzt bakterien- und pilztötende Eigenschaften. Die zur Hemmung verschiedener Bakterien- und Pilzstämme erforderlichen minimalen Hemmkonzentrationen des neuen Antibiotikums Aranorosin sind in Tabelle I angeführt.

**Tabelle I**

| Testorganismen | Minimale Hemmkonzentration (MHK) des neuen Antibiotikums Aranorosin in µg/ml |
|---|---|
| Staphylococcus aureus 209 P | 1,5 |
| Staphylococcus aureus 20204 | 1,5 |
| Staphylococcus aureus R 85 | 1,5 |
| Staphylococcus aureus SG 511 | 0,78 |
| Staphylococcus aureus 285 | 0,78 |
| Staphylococcus aureus 503 | 0,78 |
| Streptococcus faecalis | 3,0 |
| Streptococcus pyogenes 308A | 0,78 |
| Streptococcus pyogenes 77 A | 0,78 |
| Bacillus subtilis | 1,5 |
| Sarcina lutea | 1,5 |
| Pseudomonas aeruginosa ATCC 9027 | > 100 |
| Pseudomonas aeruginosa 1592 E | > 100 |
| Pseudomonas aeruginosa 1771 | > 100 |
| Pseudomonas aeruginosa 1771 M | 50 |

| | |
|---|---|
| E. coli Ese 2231 | > 200 |
| E. coli 055 | 100 |
| E. coli TEM | 100 |
| E. coli 1507 E | 100 |
| E. coli DC 0 | 100 |
| E. coli DC 2 | 25 |
| Salmonella typhimurium | 50 |
| Klebsiella aerogenes 1082 E | 50 |
| Klebsiella aerogenes 1522 E | 100 |
| Enterobacter cloacae P 99 | 100 |
| Enterobacter cloacae 1321 E | 100 |
| Candida albicans | 30 |
| Saccaromyces cerevisiae | 500 |
| Aspergillus niger | 7,5 |
| Penicillium italicum | 30 |
| Cercospora beticola | 3,0 |
| Botrytis cinerea | 30 |
| Microspora gypseum | 3,0 |

Aranorosin ist somit gegen Gram-positive und Gram-negative Bakterien sowie Hefe und fadenförmige Pilze wirksam und als Antibiotikum zur Behandlung von Bakterien- und Pilzinfektionen verwendbar.

Aranorosin hat ebenfalls cytostatische Eigenschaften und eignet sich daher als Arzneimittel zur Behandlung von Krebserkrankungen, z. B. zur Bekämpfung von malignen Tumoren und Leukämien.

## Ansprüche

1. Eine Verbindung der Formel

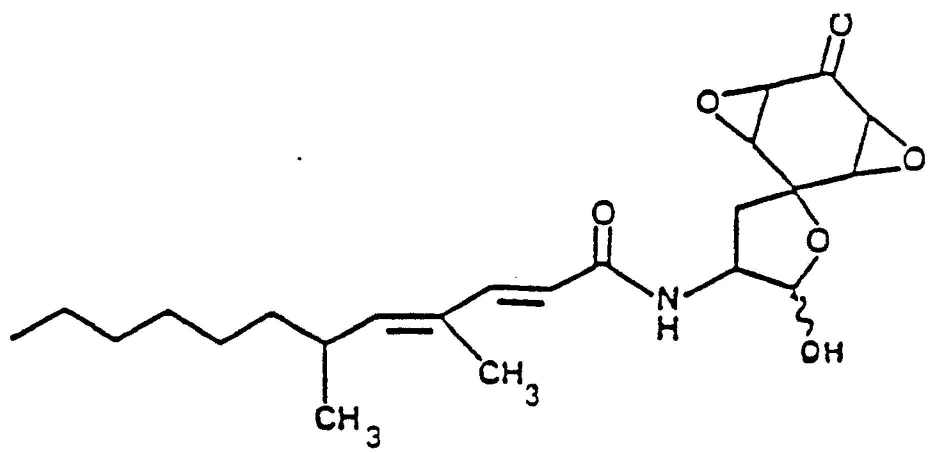

2. Verfahren zur Herstellung der Verbindung gemäß Anspruch 1, dadurch gekennzeichnet, daß man die Pilzkultur Nr. Y-30499 (DSM 4151) unter aeroben Bedingungen auf einem Nährboden, der Kohlenstoffquellen, Stickstoffquellen, anorganische Nährsalze und Spurenelemente enthält, bei einer Temperatur zwischen 24 und 30 °C und einem pH zwischen 6,0 und 8,0 66 bis 90 Stunden lang kultiviert und die Verbindung in üblicher Weise aus der Kulturbrühe isoliert und reinigt.

3. Verfahren nach Anspruch 2, dadurch gekennzeichnet, daß bei 26 °C (± 1 °C) und pH 6,5 kultiviert wird.

4. Verfahren nach Anspruch 2 oder 3, dadurch gekennzeichnet, daß die Kultivierung 72 Stunden lang durchgeführt wird.

5. Verfahren nach einem der Ansprüche 2 bis 4, dadurch gekennzeichnet, daß die Fermentation als submerse Fermentation durchgeführt wird.

6. Arzneimittel, gekennzeichnet durch einen Gehalt an einer Verbindung gemäß Anspruch 1 neben üblichen Hilfs- und/oder Trägerstoffen.

7. Verwendung einer Verbindung gemäß Anspruch 1 zur Herstellung von Arzneimitteln mit antibiotischer Wirkung.

8. Verwendung einer Verbindung gemäß Anspruch 1 zur Herstellung von Arzneimitteln mit Wirkung gegen maligne Tumore.

9. Pseudoarachniotus roseus Y-30499 (DSM 4151).

**Patentansprüche für folgende Vertragsstaaten: ES, GR**

1. Verfahren zur Herstellung einer Verbindung der Formel I

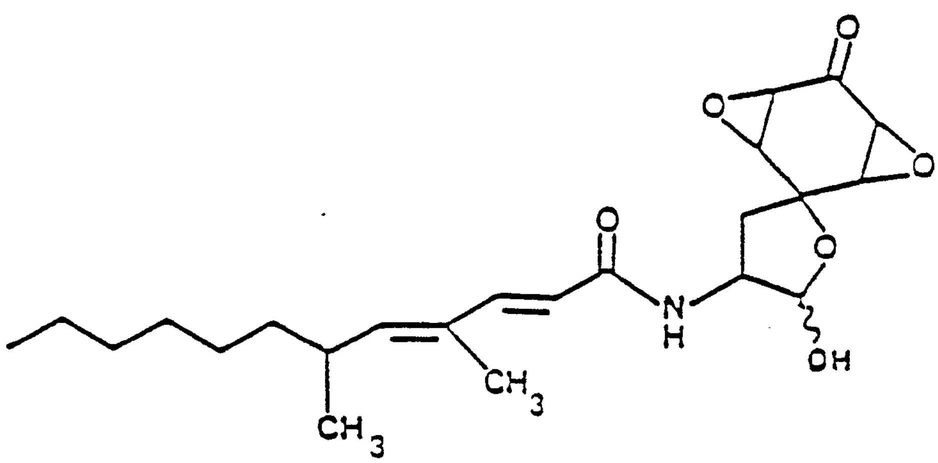

I

dadurch gekennzeichnet, daß man die Pilzkultur Nr. Y-30499 (DSM 4151) kultiviert und die Verbindung in üblicher Weise aus der Kulturbrühe isoliert und reinigt.

2. Verfahren nach Anspruch 1, dadurch gekennzeichnet, daß man die Pilzkultur unter aeroben Bedingungen auf einem Nährboden, der Kohlenstoffquellen, Stickstoffquellen, anorganische Nährsalze und Spurenelemente enthält, bei einer Temperatur zwischen 24 und 30 °C und einem pH zwischen 6,0 und 8,0 66 bis 90 Stunden lang kultiviert.

3. Verfahren nach Anspruch 1 oder 2, dadurch gekennzeichnet, daß bei 26 °C (± 1 °C) und pH 6,5 kultiviert wird.

4. Verfahren nach einem der Ansprüche 2 bis 4, dadurch gekennzeichnet, daß die Kultivierung 72 Stunden lang durchgeführt wird.

5. Verfahren nach einem der Ansprüche 2 bis 5, dadurch gekennzeichnet, daß die Fermentation als submerse Fermentation durchgeführt wird.

6. Verfahren zur Herstellung von Arzneimitteln, dadurch gekennzeichnet, daß die Verbindung der Formel I gemäß Anspruch 1 mit üblichen Hilfs- und/oder Trägerstoffen in eine geeignete Darreichungsform gebracht wird.

7. Verwendung einer Verbindung gemäß Anspruch 1 zur Herstellung von Arzneimitteln mit antibiotischer Wirkung.

8. Verwendung einer Verbindung gemäß Anspruch 1 zur Herstellung von Arzneimitteln mit Wirkung gegen maligne Tumore.

9. Pseudoarachniotus roseus Y-30499 (DSM 4151).

Europäisches Patentamt

# EUROPÄISCHER RECHERCHENBERICHT

Nummer der Anmeldung

EP 89 10 8292

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| P,X | J. AM. CHEM. SOC., Band 110, 1988, Seiten 8242-8244, American Chemical Society; H.W. FEHLHABER et al.: "Structure of aranorosin, a new antibiotic of a novel skeletal type" * Insgesamt * ----- | 1-9 | C 12 P 17/18<br>A 61 K 31/00<br>C 07 D 493/20 //<br>(C 07 D 493/20<br>C 07 D 307:00<br>C 07 D 303:00<br>C 07 D 303:00 )<br>(C 12 P 17/18<br>C 12 R 1:01 ) |
| | | | **RECHERCHIERTE SACHGEBIETE (Int. Cl.4)** |
| | | | C 12 P 17/00<br>A 61 K 31/00<br>C 07 D 493/00<br>C 12 R 1/00 |

Der vorliegende Recherchenbericht wurde für alle Patentansprüche erstellt

| Recherchenort | Abschlußdatum der Recherche | Prüfer |
|---|---|---|
| DEN HAAG | 04-08-1989 | RAJIC M. |

**KATEGORIE DER GENANNTEN DOKUMENTE**

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument